# EUROPEAN PATENT APPLICATION

(11) **EP 3 944 786 A1**
(43) Date of publication of application: **02.02.2022**
(21) Application number: 21177835.2
(22) Date of filing: 04.06.2021
(51) Int. Cl.: A46B 13/02, A46B 13/00, A46B 5/02, A46B 9/04, A45D 44/00, A61B 17/54

(54) **ABRASIVE FACIAL BUFFER SYSTEM**

(30) Priority: 27.07.2020 US 202016939861
(71) Applicant: Bailar, Lucinni, Houston, TX 77069 (US)
(72) Inventor: Bailar, Lucinni, Houston, TX 77069 (US)
(74) Representative: Loo, Chi Ching

(57) **Abstract**

An abrasive facial buffer system consists of a tool head (1), a handle (2), a vibrating mechanism (9), a power source (12), and a control switch (13). The vibrating mechanism (9) and the power source (12) are positioned within the handle (2). The control switch (13) is mounted onto the handle (2). The power source (12) is electrically connected to the vibrating mechanism (9) through the control switch (13) thus powering the tool head (1) that is operatively coupled with the vibrating mechanism (9). The tool head (1) include a skin exfoliation head that is used to remove dead skin and a pair of brush heads that is preferably utilized to clean a set of teeth with dental braces.

## Description

### FIELD OF THE INVENTION

The present invention relates generally to a facial buffer device. More specifically, the present invention is an abrasive facial buffer system that can be used to clean user's skin and teeth. The present invention further provides a set of electronics that allows for vibration of the abrasive facial buffer tip.

### BACKGROUND OF THE INVENTION

The treatment and cleaning of facial is an area of utmost importance to modern society. Face washes, treatments, abrasive rubs, soaps, masks, brushes, and a plethora of similar items and formulas are used to remove old facial cells and dirt from the external layer of facial. The use of such items leaves the user feeling refreshed and clean, and further prevents the appearances of blemishes, such as rashes, pimples, and more. Exfoliating treatments in particular have had success in cleansing the face and body. Vibrating microdermabrasion techniques in general are optimal for facial treatment, as the increased friction from gently vibrating a rough, textured surface against the facial simultaneously improves the dirt and facial removal process when compared to manually applying soaps and friction, while avoiding the inducement of pain in the user. However, such processes are limited by technology. While many buffing technologies improve the ability of the user to remove dirt and old facial, the tip of such cleaning tools often does not meet the necessary requirements. An optimal buffing tip cannot be too sharp or it will induce rashes, soreness, and undesirable redness in the target area. The tip cannot be too dull or it will not provide the ability to target particular areas of the face or facial. What is needed is a vibrating abrasive tip that is capable of targeting particular areas of the facial without damaging the facial area.

Furthermore, treatment and cleaning of teeth are important to maintain healthy set of teeth. Brushing teeth is generally considered to a good practice to maintain healthy set of teeth; however, construction of brush head extremely important to a clean set of teeth. When it comes to placement of dental braces, the existing brush heads fail to fully clean a set of teeth as they fail to reach around the dental braces.

The present invention addresses these issues with an abrasive facial buffer system that comprises a skin exfoliation head and a pair of brush heads. A handle portion of the present invention allows attachment of the skin exfoliation head and the pair of brush heads upon user's preference. The skin exfoliation head has an abrasive surface that is capable of providing the desirable friction against the facial, without being so rough as to induce pain or discomfort. The pair of brush heads provide a novel configuration of bristles so that the both brush heads can reach around dental braces to fully clean a set of teeth.

### BRIEF DESCRIPTION OF THE DRAWINGS

FIG. **1** is a perspective view of the present invention, wherein the tool head is the skin exfoliation head.
FIG. **2** is a block diagram illustrating the vibrating mechanism of the present invention and the electrical connections.
FIG. **3** is an exploded perspective view of the present invention, wherein the tool head is the skin exfoliation head.
FIG. **4** is a perspective view of the present invention, wherein the tool head is the first brush head.
FIG. **5** is a right sideview of the first brush head of the present invention.
FIG. **6** is a left sideview of the first brush head of the present invention.
FIG. **7** is a perspective view of the present invention, wherein the tool head is the second brush head.
FIG. **8** is a right sideview of the second brush head of the present invention.
FIG. **9** is a left sideview of the second brush head of the present invention.

### DETAIL DESCRIPTIONS OF THE INVENTION

All illustrations of the drawings are for the purpose of describing selected versions of the present invention and are not intended to limit the scope of the present invention.

The present invention is an abrasive facial buffer system that aids in the process of buffing the skin for exfoliating purposes and cleaning a set of teeth with dental braces. More specifically, a removably attached head of the present invention and a vibrating unit of the present invention allow the user to exfoliate skin or clean a set of teeth with dental braces.

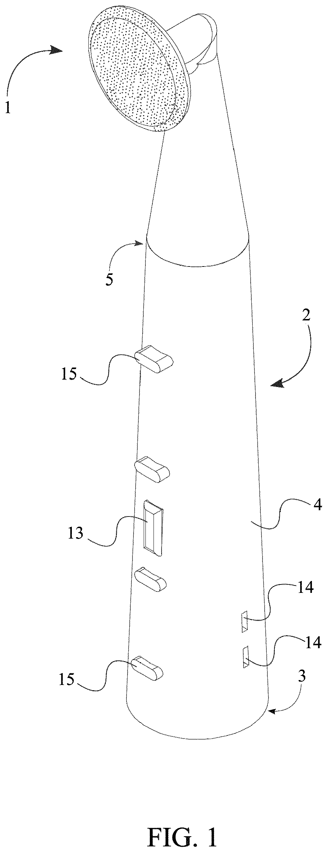

As shown in FIGS. **1-2****,** the present invention comprises a tool head **1,** a handle **2,** a vibrating mechanism **9,** a power source **12,** and a control switch **13.** The tool head **1** is used to be in contact with the skin or the set of teeth and execute the exfoliating process via vibrations that are provided by the vibrating mechanism **9.** The size and shape of the tool head **1** can vary in different embodiments of the present invention. The handle **2** is used to hold the present invention and comprises a first end **3,** a structural body **4,** and a second end **5.** The structural body **4** is extended from the first end **3** to the second end **5,** wherein the inside of the structural body **4** functions as a housing for the power source **12** and the control switch **13.** More specifically, the power source **12** is positioned within the structural body **4** to protect from outside elements. The control switch **13** is mounted onto the structural body **4** so that the user can easily access and operate the control switch **13.** The power source **12** is electrically connected to the vibrating mechanism **9** through the control switch **13,** wherein the activation of the vibrating mechanism **9** operates the tool head **1.** In other words, the tool head **1** is operatively coupled with the vibrating mechanism **9,** wherein tool head **1** is vibrated through the vibrating mechanism **9.**

In reference to FIG. **1-2****,** the shape and size of the structural body **4** can vary in different embodiments of the present invention. Preferably, the structural body **4** is shaped to be easily gripped by the user when utilizing the present thus delineating an overall cylindrical shape. In some embodiments, the structural body **4** may be tapered from the first end **3** to the second end **5.** While holding the handle **2,** the user can activate the vibrating mechanism **9** that is positioned on the structural body **4.** opposite to the handle **2.**

In reference to FIG. **1-3****,** the tool head **1** comprises a neck portion **6,** a tip receiving port **40,** and a base **30.** The size and shape of the tool head **1** can vary in different embodiments of the present invention. The tool head **1** is made of a lightweight material such as plastic so that the material properties allows the tool head **1** to maintain a degree of flexibility. Preferably, the tool head **1** is tapered body **7** and is used to orient the tool head **1** to different areas on the skin and inside the mouth. More specifically, the tip receiving port **40** is positioned within the neck portion **6** and receives vibration energy of the vibrating mechanism **9.** The base **30** is adjacently connected to the neck portion **6,** wherein the base **30** outputs the vibration energy to the user's skin or the teeth. The tip receiving port **40** is torsionally connected to the base **30** so that the outputted vibration from the vibrating mechanism **9** can be transferred to the base **30** via the tip receiving port **40.**

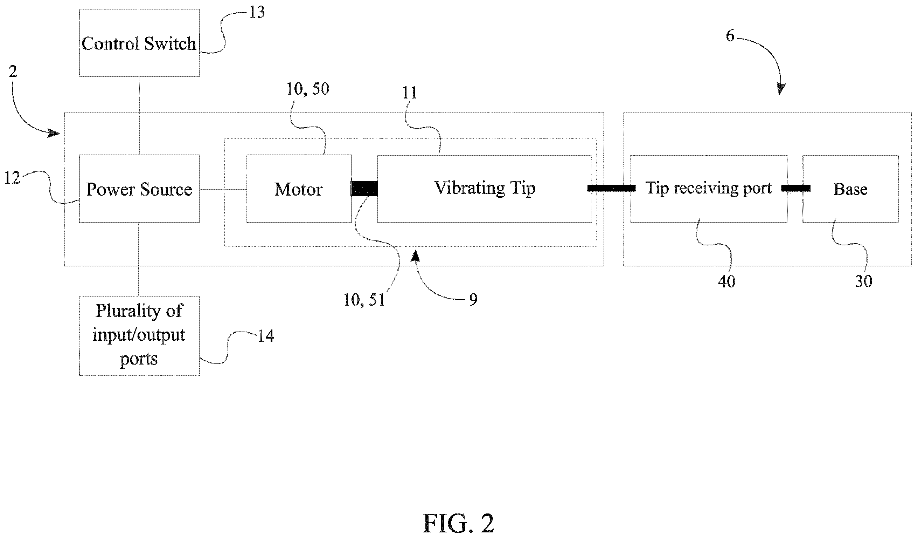

In reference to FIG. **2****,** the vibrating mechanism **9** comprises a motor **10** and a vibration outputting tip **11.** More specifically, a rotor **51** of the motor **10** is torsionally connected to the vibration outputting tip **11** while a stator **50** of the motor **10** is internally connected to the structural body **4.** The motor **10** is electrically connected to the power source **12** through the control switch **13.** The positioning of the control switch **13** allows the user to promptly activate or deactivate the vibrating mechanism **9** and hence, the vibrations applied to the skin or teeth. To do so, the control switch **13** is electrically connected in between the power source **12** and the motor **10.** Additionally, the control switch **13** can be configured to control the vibration frequency of the vibrating mechanism **9.** Different frequencies can be beneficial to exfoliate skin in different areas of the body or clean teeth. For an example, a higher vibration frequency can be used to exfoliate skin on the foot. On the other hand, a lower vibration frequency can be used to exfoliate skin on the face.

In the preferred embodiment, the power source **12** is a rechargeable battery. However, the power source **12** can vary in different embodiments of the present invention. For example, in some embodiments the power source **12** may be a power cord that can be plugged into a wall outlet or power adapter. To provide the necessary power to the vibrating mechanism **9,** the power source **12** is electrically connected to the motor **10** as illustrated in FIG. **2****.** Once the motor **10** is activated through the control switch **13,** the motor **10** receives electrical power from the power source **12.** Next, the motor **10** activates the vibration outputting tip **11** by being mechanically or torsionally engaged with the rotor **51** is the motor **10.** To position the power source **12** distant from the tool head **1,** and to maintain an aesthetically pleasing appearance, the power source **12** is positioned within the structural body **4.**

As previously discussed, the power source **12** can be, but is not limited to, a rechargeable battery. To recharge the power source **12,** the present invention further comprises at least one input port **14** that traverses into the structural body **4.** To execute charging functionalities, the at least one input port **14** is electrically connected to the power source **12.**

When utilizing the present invention, maintaining a firm grip is essential. To aid with the process, the present invention further comprises a plurality of gripping portions **15** as shown in FIG. **1-3****.** The plurality of gripping portions **15** is externally distributed along the structural body **4** in between the first end **3** and the second end **5.** Resultantly, the rough texture of the plurality of gripping portions **15** provides a non-slip holding area for the user.

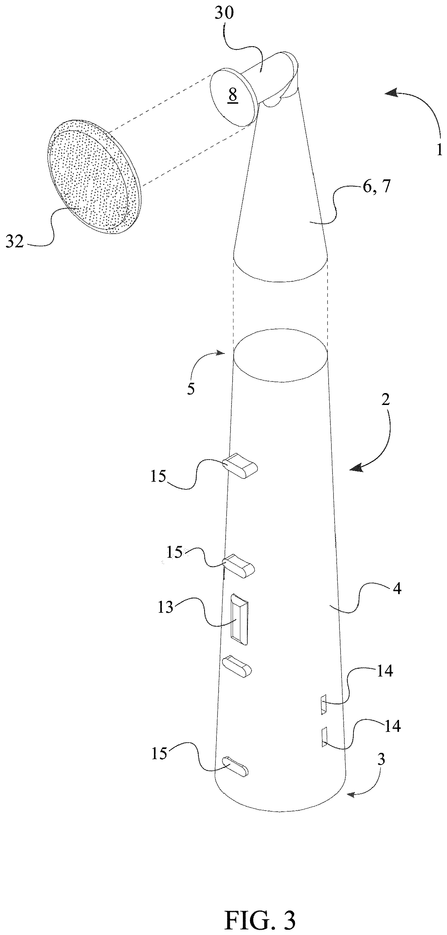

In one embodiment of the tool head **1** that functions as a skin exfoliation head as shown in FIG. **3****.** The present invention further comprises an abrasive pad **32.** The abrasive pad **32** is superimposed over a contact surface **8** of the base **30** so that the tool head **1** can be used to remove an outer layer of dead skin in a preferred area. The abrasive pad **32** is generally shaped similar to the shape of the contact surface **8** so that only the abrasive pad **32** is in contact with the user's skin. Preferably the contact surface **8** is circular and to accommodate the circular shape of the contact surface **8,** the abrasive pad **32** is also circular in shape in the preferred embodiment of the present invention. However, the size and shape of the contact surface **8** can vary in different embodiments of the present invention. Furthermore, in some embodiment, the abrasive pad **32** is removably mounted to the contact surface **8** thus allowing only the abrasive pad **32** to be changed according the user's preference. In some embodiment, the abrasive pad **32** is connected to the contact surface **8** wherein the abrasive pad **32** and the base **30** complete a permanent connection between the said components. Removability of the tool head **1** or the abrasive pad **32** is beneficial if the user intends on utilizing different textures of the tool head **1.** In one instance, the user might prefer the tool head **1** to be rough. In another instance, the user might prefer the tool head **1** to be soft.

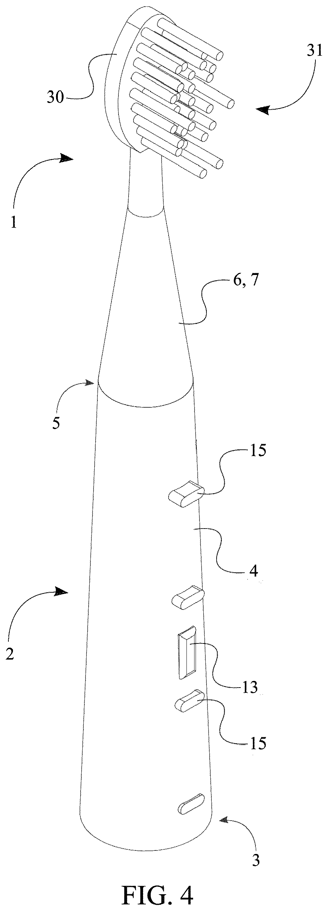

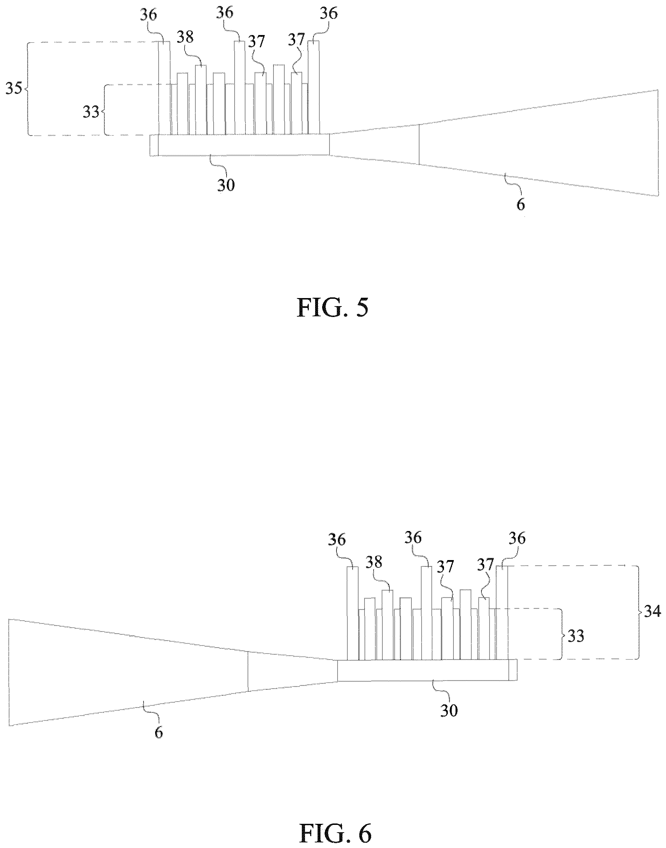

In one embodiment of the tool head **1** that functions as a first brush head to clean around the dental braces as shown in FIG. **4-6****.** The present invention further comprises a plurality of bristles **31** that is connected to the base **30.** Furthermore, the plurality of bristles **31** comprises a set of inside bristles **33,** a set of left outer bristles **34,** and a set of right outer bristles **35.** More specifically, the set of inside bristles **33** is encircled by the set of left outer bristles **34** and the set of right outer bristles **35** thus maximizing the clean abilities around the dental braces. In reference to FIG. **5-6****.** a height of the set of inside bristles **33** is preferably 6.5 millimeters (mm) and represent the shortest bristles of the corresponding brush head. The set of left outer bristles **34** and the set of right outer bristles **35** each comprises at least one tall bristle **36,** at least one short bristle **37,** and at least one intermediate bristle **38.** More specifically, the intermediate bristle **38** is interspaced between a pair of short bristles **37.** The intermediate bristle **38** and the pair of short bristles **37** are interspaced between a pair of tall bristles **36.** In order to reach around the dental braces, a height of the tall bristle **36** is preferably 12 mm, a height of the short bristle **37** is preferably 8 mm, and a height of the intermediate bristle **38** is preferably 9 mm.

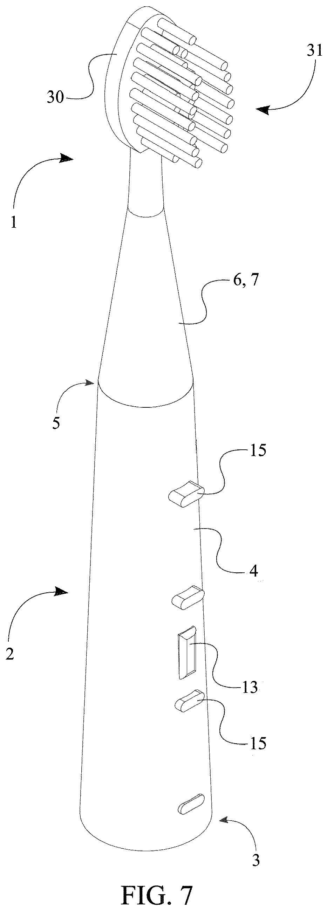

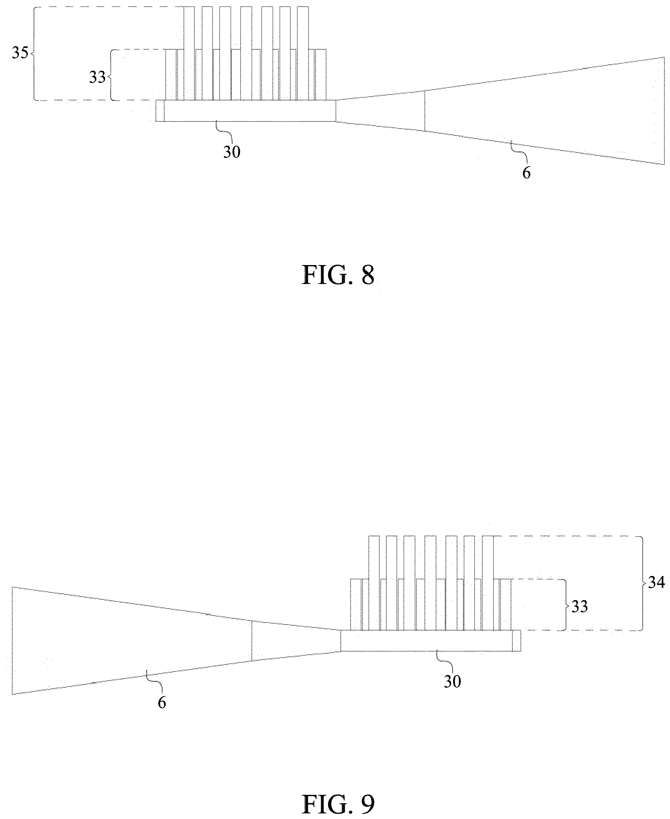

In one embodiment of the tool head 1 that functions as a second brush head to clean around the dental braces as shown in FIG. **7-9****.** The present invention further comprises the plurality of bristles **31** that is connected to the base **30.** Furthermore, the plurality of bristles **31** comprises the set of inside bristles **33,** the set of left outer bristles **34,** and the set of right outer bristles **35.** More specifically, the set of inside bristles **33** is interspaced within the set of left outer bristles **34** and the set of right outer bristles **35** thus maximizing the clean abilities around the dental braces. In reference to FIG. **8-9****.** a height of the set of inside bristles **33** is preferably 7 mm and represent the shortest bristles of the corresponding brush head. The set of left outer bristles **34** and the set of right outer bristles **35** each comprises identical sized bristles as the height of the set of left outer bristles **34 is** 12 mm and a height of the set of right outer bristles **35** is 12 mm.

The following process flow is generally followed when utilizing the present invention. Initially, the user holds the present invention by the handle **2** and orients the tool head **1** so that the tool head **1** is at the preferred area on the skin or within the mouth. When the tool head **1** is positioned as preferred, the user activates the vibrating mechanism **9** via the control switch **13.** The frequency of the vibrations can also be adjusted via the control switch **13.** In the preferred embodiment of the present invention, 30,000-40,000 vibrations are produced per minute. Next, by pressing the tool head **1,** the exfoliating process or the cleaning process can be executed. When the tool head **1** is worn out, the old tool head **1** can be replaced with a newer tool head **1** according to user preference. When the power source **12** need to be recharged, the user utilizes the at least one input port **14** to recharge the power source **12.** By utilizing the skin exfoliation head of the present invention, wrinkles can be reduced, pores can be minimized, acne scars can be reduced, elasticity of the skin can be improved, stretch marks can be reduced, and uneven skin tones can be improved. By utilizing the brush head of the present invention, gumline, teeth, cavities, gaps, and around dental braces can be efficiently cleaned thus improving the overall health of the teeth.

Although the invention has been explained in relation to its preferred embodiment, it is to be understood that many other possible modifications and variations can be made without departing from the spirit and scope of the invention as hereinafter claimed.

## Claims

1. An abrasive facial buffer tip comprising:
a tool head;
a handle;
a vibrating mechanism;
a power source;
a control switch;
the handle comprising a first end, a structural body, and a second end;
the structural body extending from the first end to the second end;
the vibrating mechanism being positioned within the structural body;
the power source being positioned within the structural body;
the control switch being mounted onto the structural body;
the power source being electrically connected to the vibrating mechanism through the control switch; and
the tool head being operatively coupled with the vibrating mechanism, wherein tool head is vibrated through the vibrating mechanism.

2. The abrasive facial buffer tip as claimed in claim 1 comprising:
the tool head comprising a neck portion, a tip receiving port, and a base;
the tip receiving port being positioned within the neck portion;
the base is adjacently connected to the neck portion; and
the tip receiving port being torsionally connected to the base.

3. The abrasive facial buffer tip as claimed in claim 2, wherein the neck portion is a tapered body.

4. The abrasive facial buffer tip as claimed in claim 1 comprising:
the vibrating mechanism comprises a motor and a vibration outputting tip;
a rotor of the motor being torsionally connected to the vibration outputting tip;
a stator of the motor being internally connected to the structural body; and
the motor being electrically connected to the power source through the control switch.

5. The abrasive facial buffer tip as claimed in claim 1 comprising:
at least one input port;
the at least one input port traversing into the structural body; and
the at least one input port being electrically connected to the power source.

6. The abrasive facial buffer tip as claimed in claim 1 comprising:
a plurality of gripping portions; and
the plurality of gripping portions being externally distributed along the structural body between the first end and the second end.

7. The abrasive facial buffer tip as claimed in claim 1 comprising:
an abrasive pad;
the tool head comprising a base; and
the abrasive pad being superimposed over a contact surface of the base.

8. The abrasive facial buffer tip as claimed in claim 7, wherein the abrasive pad is removably mounted to the contract surface.

9. The abrasive facial buffer tip as claimed in claim 7, wherein the contact surface is circular in shape.

10. The abrasive facial buffer tip as claimed in claim 7, wherein the abrasive pad is circular in shape.

11. The abrasive facial buffer tip as claimed in claim 1 comprising:
a plurality of bristles;
the tool head comprising a base;
the plurality of bristles being adjacently connected onto the base;
the plurality of bristles comprising a set of inside bristles, a set of left outer bristles, and a set of right outer bristles; and
the set of inside bristles being encircled by the set of left outer bristles and the set of right outer bristles.

12. The abrasive facial buffer tip as claimed in claim 11, wherein a height of the set of inside bristles is 6.5 mm.

13. The abrasive facial buffer tip as claimed in claim 11 comprising:
the set of left outer bristles and the set of right outer bristles each comprises at least one tall bristle, at least one short bristle, and at least one intermediate bristle;
the intermediate bristle being interspaced between a pair of short bristles;
the intermediate bristle and the pair of short bristles being interspaced between a pair of tall bristles;
a height of the tall bristle being 12 mm;
a height of the short bristle being 8 mm; and
a height of the intermediate bristle being 9 mm.

14. The abrasive facial buffer tip as claimed in claim 1 comprising:
a plurality of bristles;
the tool head comprising a base;
the plurality of bristles being adjacently connected onto the base;
the plurality of bristles comprising a set of inside bristles, a set of left outer bristles, and a set of right outer bristles; and
the set of inside bristles being interspaced within the set of left outer bristles and the set of right outer bristles.

15. The abrasive facial buffer tip as claimed in claim 14 comprising at least one of the following;
wherein a height of the set of inside bristles is 7 mm;
wherein a height of the set of left outer bristles is 12 mm; and
wherein a height of the set of right outer bristles is 12 mm.
